# EUROPEAN PATENT APPLICATION

(11) **EP 3 202 370 A1**
(43) Date of publication of application: **09.08.2017**
(21) Application number: 16382044.2
(22) Date of filing: 02.02.2016
(51) Int. Cl.: A61F 2/16

(54) **SUPPORTING DEVICE FOR THE INSERTION OF AN INTRAOCULAR LENS**

(71) Applicant: Fundacion Tekniker, 20600 Eibar (Guipuzkoa) (ES); Palomino Munoz, Antonio, 28009 Madrid (ES)
(72) Inventor: PALOMINO MUÑOZ, Antonio, 28009 Madrid (ES); OÑATE GUTIERREZ, Roberto, 20600 Eibar, Guipúzcoa (ES)
(74) Representative: Balder IP Law, S.L.

(57) **Abstract**

A device (50) to aid the insertion of an intraocular lens (10) in an intraocular cavity without a capsular bag or sufficient capsular remnants to support an IOL, wherein said intraocular lens (10) comprises two haptics (12). The device (50) comprises: a piece (53) manufactured from a biocompatible material, wherein the piece (53) is configured to receive a haptic (12) of the lens (10); and a thread (54) made of a biocompatible material attached at one end (52) of said piece (53), said thread (54) being configured to, in use of the device, pull and position the optical part (11) of the lens (10) inside the intraocular cavity. An ensemble made up of two devices like the one indicated above and a lens with two haptics. Each haptic is anchored to a support device. Use of the aforementioned device or ensemble in a surgical procedure. A procedure for positioning an intraocular lens (10) comprising two haptics (12) in an intraocular cavity of a patient lacking a capsular bag or capsular remnants to support the IOL, using two support devices (50).

## Description

### TECHNICAL FIELD

This invention pertains to the field of medical devices and, specifically, to intraocular lenses and devices for the insertion and positioning of intraocular lenses.

### BACKGROUND OF INVENTION

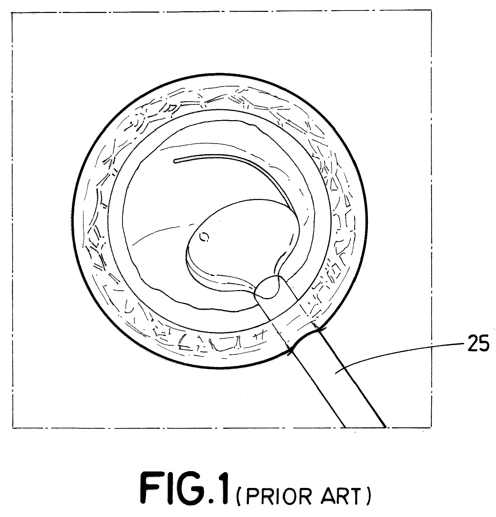

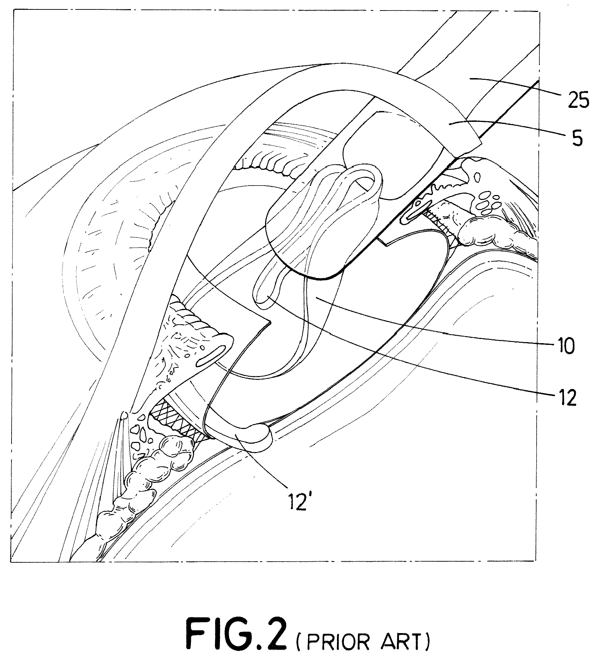

In modern cataract surgery, the entire procedure is performed through a small corneal incision (the incision is normally approximately 2.70 mm long, but can be as small as 1.8 mm). Phacoemulsification is used to extract all but the posterior capsule of the cataract. Then, a folded intraocular lens (IOL) is inserted; this lens is going to be lodged in that posterior capsule with its bag, as illustrated in Figures 1 and 2. Figure 2 presents the prescribed procedure in the case of capsule and bag integrity, for the insertion of the IOL through the corneal incision 5 using an applicator or injector 25 in which the IOL 10 has been previously inserted.

Some eye conditions, such as congenital anomalies, complicated cataract surgery, eye trauma, etcetera, can result in the patient having to undergo IOL insertion but in the absence of capsular bag integrity or anterior capsular remnants for the secure attachment of the IOL. In these situations, the possible alternatives are: (1) Use of an anterior chamber IOL: Some difficulties of this option include that the anterior chamber is not the natural site for an intraocular lens and, moreover, it requires large corneal incisions, which bring with them a risk of astigmatism and corneal decompensation. (2) Use of an iris claw IOL: As in the previous case, they are implanted in an unnatural site like the anterior chamber and require a large corneal incision. (3) Use of an iris suture-fixated IOL: The difficulties of this option include that the surgery is very laborious and the lens can luxate over time due to dehiscence of the sutures. (4) Use of a sulcus suture-fixated IOL: These lenses tend to be rigid, thereby requiring a large corneal incision, cause astigmatism and involve a risk of dehiscence of the sutures with luxation of the lens. (5) Use of an IOL with haptics buried in the scleral tunnel. This option is considered the most advantageous, since the IOL is foldable, thereby only requiring a small incision. It is positioned in the posterior chamber with no sutures.

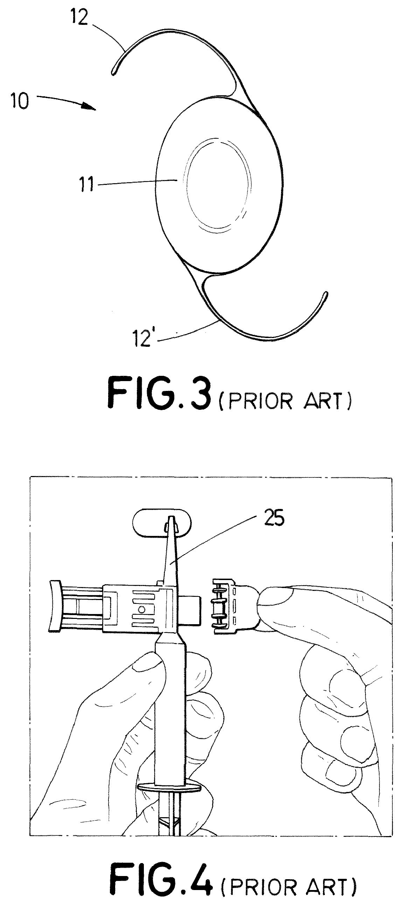

Figure 3 presents a conventional IOL 10 with haptics. This lens is made up of an optical part 11, which is the part of the lens that takes the light to the retina, and by a haptic part 12 made up of two filaments 12 that are connected to the optical part 11 of the IOL that serve to keep the IOL in the eye. The two filaments 12 are also known as "haptics", and this type of IOL is known as a 3-piece IOL.

Lenses like those shown in Figure 3 are inserted through a small incision in the cornea, normally using an applicator or injector. Figure 4 presents a possible system used regularly to insert the IOL in an applicator or injector. The IOL is folded for insertion. The main difficulty of the insertion procedure of the IOL through the corneal incision is that, as the lens is inserted and unfolds inside the eye, in which there is no crystalline capsule, we have to grasp the haptic first with a clip and then extract it from the eyeball through an incision in the sulcus. During this manoeuvre, there is a relatively high risk of the IOL detaching from the applicator during the insertion in the eye and not being able to grasp it with the clip or even of the haptic breaking and detaching from the optical part during the manoeuvre to extract the haptic from the eye. This puts the patient's safety at risk, since the IOL would luxate to the back of the eye and the surgeon would have to find the luxated lens in the eye and try to recover it with the help of clips or other surgical elements in a later, unscheduled vitrectomy. The luxated lens in the back of the eye can be a major risk depending on the surgeon's experience. It has to be elevated to the level of the iris to be able to re-implant it in its proper position. The intraocular manoeuvres required to do this can result in retinal lesion and cause retinal detachment.

Moreover, a strut of the lens may break, in which case it would have to be replaced. It is also important to keep in mind that the lens was inserted folded, and once it unfolds, it cannot be removed through the original corneal incision. Therefore, it has to be cut using scissors, which involves a greater chance of iris and corneal lesions.

### INVENTION DESCRIPTION

The present invention aims at resolving the aforementioned difficulties using a device to facilitate the insertion and positioning of an intraocular lens (IOL) and to prevent the luxation of the IOL to the vitreous chamber and back of the eye, as well as the rupture of its haptics. The use of this device drastically reduces the risks inherent to the surgery for inserting these lenses. As indicated above, the device is made of a piece to crimp or anchor the end of one haptic and a thread attached or anchored to the previous piece. The device is designed to enter through the corneal incision and exit through the sulcus of the eye during the insertion surgery, thereby indicating the path to be followed by the haptic of the IOL. The device therefore reduces the surgeon's stress, and enables him or her to perform the procedure much faster and much more safely.

In the first embodiment of the invention, it is provided a device to aid the insertion of an intraocular lens in an intraocular cavity without a capsular bag or sufficient capsular remnants for lens fixation, wherein said intraocular lens is made up of one optical part and two haptics. The device comprises: a piece manufactured with a biocompatible material, wherein the piece is configured to receive a portion of the haptic in its interior through the first end of the piece; and a thread made of biocompatible material attached to the end of said piece located opposite the end designed to receive the haptic. The thread, when this device is in use, is configured to pull and position the optical part of the lens inside the intraocular cavity.

In one possible embodiment, the cross-section of the thread ranges between 0.030 and 0.350 mm in diameter.

In one possible embodiment, the piece has an outer length of no greater than 2 mm.

In one possible embodiment, the part of the piece designed to receive a part of the haptic has an interior length of no greater than 1 mm.

In one possible embodiment, the piece is metallic.

In one possible embodiment, the piece, is a tube designed to, in use of the device, crimp a portion of the haptic.

In one possible embodiment, the exterior diameter of the piece ranges between 0.250 and 0.700 mm.

In one possible embodiment, the interior diameter of the piece ranges between 0.200 and 0.430 mm.

In one possible embodiment, the device has a rivet where the thread and the base of the piece join to reinforce the connection between the thread and the piece.

In another embodiment of the invention, it is provided an ensemble comprising: a first device to aid the insertion of an intraocular lens in an intraocular cavity in the absence of a capsular bag or sufficient capsular remnants for lens fixation, as described above, and an intraocular lens that comprises an optical part, a first haptic and a second haptic; in which a portion of the free end of the first haptic is designed to be inserted and anchored in the piece of the first device.

In one possible embodiment, the ensemble also comprises a second device to aid the insertion of an intraocular lens in an intraocular cavity in the absence of a sufficient capsular bag or capsular remnants for lens fixation, as explained above. One portion of the free end of the second haptic is designed to be inserted and anchored in the piece of the second device.

In another embodiment of the invention, a use of the device or ensemble described above in a surgical procedure is provided.

In another embodiment of the invention, the aforementioned device or ensemble is provided, for use in a surgical method involving the insertion and positioning of an intraocular lens in a patient lacking a capsular bag.

In the last embodiment of the invention, a procedure is provided to position a 3-piece, foldable intraocular lens comprising an optical part and two haptics in an intraocular cavity of a patient in the absence of a capsular bag or sufficient capsular remnants for lens fixation, using two devices like those described above (one for each haptic). The procedure comprises: Insert a portion of the free end of the first haptic of that lens in a cavity defined by a first device, while the optical part of the lens is folded in an injector; close or deform the end of the device defining that cavity until the haptic is anchored to the device; Insert the free end of the thread of the first device through an incision made in a cornea and take the free end of the thread through and out of the sulcus (the IOL is still outside of the eye); with the help of the injector, insert that lens in the ocular cavity through the incision made in the cornea, so that said free end of the thread remains outside of the patient and, by pulling on it, extract the first haptic until it is outside the eye, while the second haptic remains outside of the eye; insert a portion of the free end of the second haptic of that lens in a cavity defined by a second device, while this second haptic is outside of the eye and the optical part of the lens has already been injected inside the eye; close or deform the end of the second device defining that cavity until the second haptic is anchored to the second device; insert the free end of the thread of the second device through the incision made in the cornea and take the free end of the thread through and out of the sulcus; Insert the second haptic in the ocular cavity with the help of a clip while pulling the thread connected to the second haptic from outside the eye, so that the second haptic is extracted from the ocular cavity, following the thread, until it is outside of the eye; place the optical part of the lens in the desired position in the intraocular cavity by manoeuvring the two threads connected to the respective haptics from outside of the patient and affix both haptics to their respective lodging.

Thanks to the device and its connection to the haptic of the lens, the surgeon avoids having to grasp each haptic of the lens situated inside the eye with clips and having to move them to their lodging, and therefore also prevents the lens from slipping to the back of the eye. With conventional methods, when clips are used to hold the haptics, they can quickly slip through the clip, causing the lens to fall to the back of the eye. Therefore, risks of eye lesions and of surgical complications are avoided since the surgeon clips the thread of the device and removes it from the eye through an incision in the sulcus without the IOL having been inserted in the eye. This relieves the surgeon of stress and provides confidence that the IOL is not going to luxate to the back of the eye since the haptic is going to follow the path of the thread and because there will be no sharp or sudden manoeuvres with the clips that would break the haptics.

The advantages of the invention will be more apparent given the descriptions provided below.

### BRIEF DESCRIPTION OF THE SKETCHES

To complement the description and with the aim of helping to better understand the invention's characteristics in accordance with a practical example of embodiment, the following figures are enclosed as an integral part of the description. This set of figures is not restrictive and must be used as examples only, as follows:
Figures 1 and 2 depict the insertion of an IOL, inserted in an applicator or injector, through an incision made in the cornea in a surgical procedure free of complications and in which the eye has a capsular bag.
Figure 3 depicts a scheme of a conventional intraocular lens (IOL) with haptics.
Figure 4 depicts a possible system used regularly for inserting an IOL in an applicator or injector.
Figure 5A illustrates a device for the insertion of an IOL in the ocular cavity in accordance with a possible embodiment of the invention. Figures 5B and 5C depict a longitudinal cross-section of the device in accordance with a possible embodiment of the invention.
Figures 6A and 6B each present schemes of an ensemble made up of an IOL and a device like that in Figures 5A to 5C.

### DESCRIPTION OF ONE MODE OF THE INVENTION'S EMBODIMENT

In this text, the word "comprise" and its variations (like "comprising", etc.) shall not be interpreted as excluding the possibility that the description herein may include other elements, stages, etc.

In the context of this invention, the term "approximately" and its family of terms (such as "approximate", etc.) shall be understood as indicative values very close to those accompanying the previously mentioned term. That is to say, deviations from an exact value should be accepted within acceptable limits, since the expert in the technique will understand that said deviation from the indicated values is inevitable due to the imprecision of measurement, etc. The same applies to the terms "around" and "substantially".

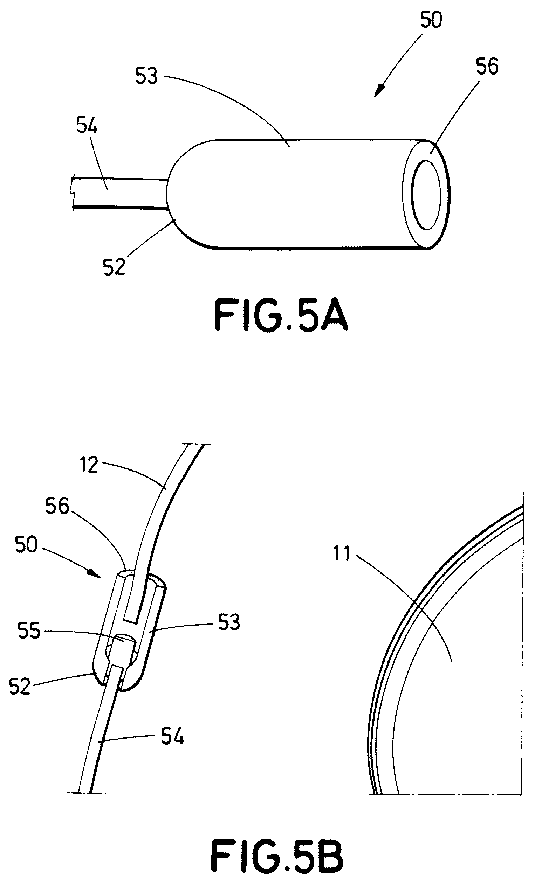

Figure 5A illustrates a device 50 for the insertion of an IOL in the ocular cavity in accordance with a possible embodiment of the invention. The device 50 is made up of a piece 53 and a suture thread 54. The piece 53 is preferably a small tube, nozzle, or short, hollow, blunt surgical needle. Piece 53, preferably a tube, forms a base on one of its ends 52. The base can be flat, tronchoconic (conical frustrum shape), semi-spherical, or any other shape. Preferentially, a shape is selected that does not hinder the manipulation of the device by the surgeon or medical personnel.

The piece 53, which is hollow, acts as a crimper or connection for one haptic of the IOL, as explained below. That is, piece 53 can be considered crimping means. One device 50 is used per haptic. Piece 53 is made of a biocompatible material. As an example, which is not limitative, a metal is used, such as stainless steel, which could be martensitic stainless steel (the so-called high-alloy stainless steels with chromium and other elements. They provide good resistance to corrosion and mechanical resistance and are magnetic). In the event that the device detaches and falls to the back of the eye due to an error during use, a magnet can be used to recover it, thereby guaranteeing its extraction.

The base 52 has a suture thread 54 attached thereto that connects to the base 52 in a conventional way. The connection between the thread 54 and the base 52 of the crimper can be manufactured in different ways. As an example, thread 54 is threaded or attached as they are conventionally to suture needles. The thread 54 attached or threaded to the base 52 is made of a biocompatible material. A thread with certain rigidity, such as nylon or polypropylene, is selected preferably, but not in a limiting way. The cross-section or thickness of the thread is of a 5/0 calibre or smaller. The suture thread calibre indicates the diameter of the suture material. It is measured numerically and its enumeration has been defined by United States Pharmacopeia (USP). For example, 5/0 indicates "5 zeros" (the greater the number of zeros, the smaller the diameter). The cross-section of the thread 54 ranges between 0.030 and 0.350 mm in diameter. Preferably, the thread 54 has a length ranging between 1 and 5 cm.

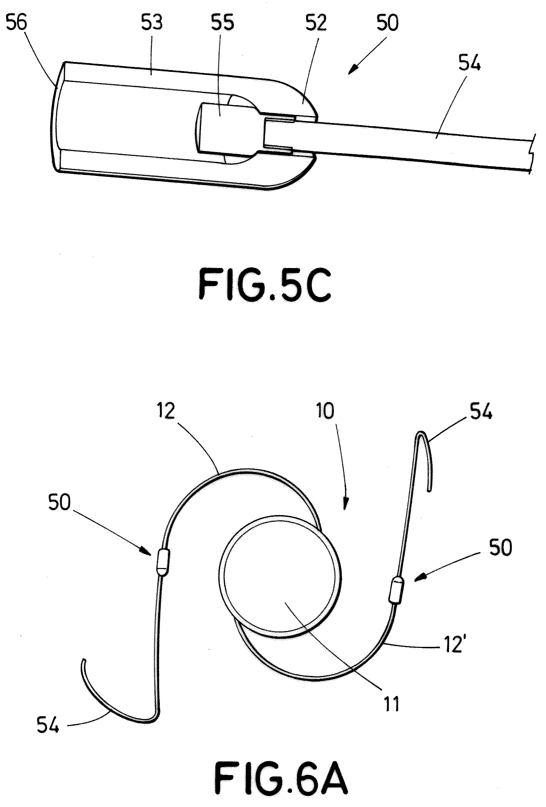

Figure 5B presents a longitudinal cross-section of the device 50 in accordance with a possible embodiment, depicting the base 52 in detail, to which the end of the thread 54 attaches or is threaded. In this embodiment, the cross-section of the tube forming the piece 53 narrows or tapers in size in the base 52, such that the cross-section of the piece 53 in the area where the thread 54 attaches or is threaded is a similar size (equal to or slightly larger than) to the cross-section of the thread 54. Therefore, the thread 54 is prevented from easily detaching from the piece 53. Also depicted is how a portion of the free end of the haptic 12 12' is inserted in the free end 56 of the piece (tube) 53. Finally, also depicted is a portion of lens 11 that is connected, though not shown, to the haptics 12 12'. Figure 5C presents a longitudinal cross-section of the device 50. In the figure, the free end of the haptic has been omitted. The piece 53, including its base 52, has a length of no greater than 2 mm, preferably no longer than 1 mm. The space defined in the cavity of the tube forming the piece 53 is designed to hold a portion of the free end of a haptic 12 12' of the IOL. The depth of the interior of the tube (dimension throughout the longitudinal axis of the tube) is no greater than 1 mm, and preferably no greater than 0.6 mm. The interior diameter of the tube 53 ranges from 0.190 to 0.430 mm, preferably between 0.200 and 0.300 mm. The diameter of the haptic 12 12' is usually 0.140 mm. The exterior diameter of the tube 53 (and also the interior) depends on the thickness of the thread 54 selected. In a preferred embodiment, the exterior diameter of the tube 53 ranges between 0.250 and 0.700 mm, more preferably between 0.350 and 0.650 mm. As indicated, the cross-section of the thread 54 ranges from 0.030 to 0.350 mm in diameter, so, obviously, in those cases in which a thread with a diameter of 0.350 mm is used, the exterior diameter of the tube will be greater than 0.350 mm. Also, a tube 53 with an exterior diameter of 0.250 mm can be used if the thread 54 has a diameter that is less than 0.250 mm. Logically, the thread diameter and the exterior tube 53 diameter values should be consistent with the values for the interior diameter of the tube 53, and must be greater than or equal to the diameter of the thread and, of course, less than the exterior diameter of the tube.

Once the thread 54 is attached or threaded to the base 52 of the piece (tube) 53 of the device 50, the other end of the thread 54 is left free, so that it can be used to pull the ensemble if necessary. Preferably, each thread is connected to the base 52 industrially. In a possible embodiment, device 50 includes, at the point of union between the thread 54 and the base 52 of the piece 53, a closure or rivet 55 to reinforce the connection (to connect, the same technology that is currently used for the connection of a thread to a surgical needle is used) and prevents the release of the thread, illustrated in Figure 5B. Preferably, the closure or rivet 55 is metallic. The closure or rivet 55 is preferably situated inside the piece 53 and proximal to the base 52 through which the thread 54 enters.

In an alternative embodiment that is not illustrated, the base 52 of the piece 53 is completely closed, in which case the thread 54 is crimped to the piece 53 without penetrating the inside of the tube forming the piece 53. Conventional techniques are used for this crimping.

Before inserting the lens with the injector through the cornea, the free end of the first haptic of the IOL is inserted in the cavity defined in the device 50 (specifically, in the tube 53). This is done once the lens is folded in the injector and the first haptic 12 emerges. At that time, the end of the haptic 12 is crimped in the piece 53 (see Figure 5B). In a preferred embodiment, it is inserted in the cited cavity defined by the piece 53 between 0.40 and 0.60 mm from the end of the haptic. More preferably, 0.5 mm of the end of the haptic 12 is inserted. As stated, the depth of this space is no greater than 1 mm, preferably, it is no greater than 0.6 mm. After inserting the end of the haptic 12 in the cavity, the surgeon flattens, preferably using a medical device, such as a needle holder, the part of the tube 53 inside of which the end of the haptic 12 has been inserted, until the end of the haptic 12 is anchored to or captured in the flattened tube 53, which tractions the haptic 12 so that it does not detach from the device 50. As indicated, the depth (distance throughout the longitudinal axis) of the hollow part (interior of the tube) is no greater than 1 mm, preferably no greater than 0.6 mm. That is, the cylindrical wall of the tube defining the cavity is flattened, until the end of the haptic is anchored or captured between the flattened, deformed walls of the tube, which traction the haptic so that it does not detach from the device 50. Once the first haptic 12 is removed and the lens, already injected, remains in the corneal wound, the second haptic 12' is crimped in a second piece 53 (of a second device 50, similar to that above) and the process from the first haptic is repeated, but with the IOL already inside the eye. Alternatively to the attachment of the portion of haptic 12 (or 12') to the piece 53 using traction or flattening of the part of the piece 53 through which the haptic has been inserted, the device 50 can incorporate attachment means, not depicted, that prevent, once a portion of haptic 12 has been inserted in the cavity of the piece 53, the portion of the haptic inserted from moving backwards and detaching from the device 50. As an example, piece 53 comprises, inside the cavity, at the level of the end 56 through which a portion of haptic is inserted, a plurality of retractile elements, such as inverted teeth, not depicted, that gently advance as the haptic pushes them in its advancement, and as the haptic stops, they move backwards slightly to attempt to recover their original position, thereby capturing the haptic, which will not be able to exit.

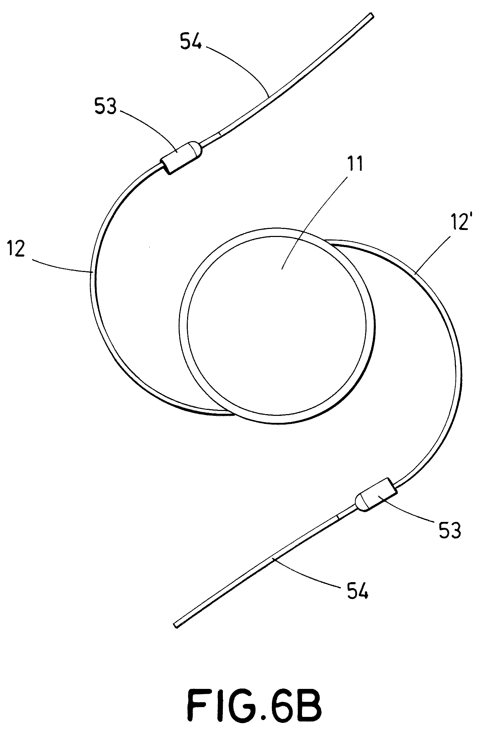

Figures 6A and 6B present a scheme for an ensemble made up of a device according to the invention connected to a conventional lens with haptics. The illustration depicts the lens 10, comprising the optical part or lens itself 11 and two filaments or haptics 12 12'. The end of each haptic 12 12' has been crimped or connected to a respective device 50 made up by the piece 53 and the thread 54.

The use of the ensemble in a surgical procedure to implant, in the eye of a patient, an intraocular lens (IOL) that has been mounted in an injector, is as follows:
1- Insert the end of a haptic 12 of the IOL 10 in the cavity defined in the device 50 and flatten or deform the end of the device 50 defining that cavity until the haptic 12 is anchored to the device 50. Alternatively, use a device 50 with attaachment means, for example, retractile elements that prevent the haptic from detaching from the device 50. The lens is already mounted -folded- in the injector.
2- Then insert the end of the thread 54 through a corneal incision and thread it out of an incision in the sclera at the level of the sulcus with the aid of a clip. This manoeuvre, since the lens 10 is still outside of the eye and there is no danger of it falling into the vitreous cavity, relieves a certain amount of stress for the surgeon, who, without the device 50, would have to grasp the haptic 12 of the lens 10 with clips from the sulcus when the lens 10 is already inside the eye and with a relatively high probability of it falling to the vitreous cavity.
3- Insert the lens 10 previously inserted in an injector/applicator through an opening made in the cornea. To do this, the injector is inserted inside the eye through the corneal incision and the lens is injected. Once the injection of the lens into the eye is completed, the optical part remains inside the eye and the first haptic has been extracted through the sulcus connected to the thread (haptic 12 follows the thread of device 50, so that when the thread is pulled from outside the eye, haptic 12 exits the eye). The second haptic remains, for the time being, outside the eye in the corneal incision.
4- Since the other haptic 12' of the lens 10 is still outside of the eye, point 1 is repeated for this second haptic 12'. That is, the second haptic is crimped to a second device 50. Then, the end of thread 54 of the second device 50 is inserted through the corneal incision and we thread it out of an incision in the sclera at the level of the sulcus with the aid of a clip used to pull the thread. The scleral incisions at the sulcus level are located approximately 180º from one another.
5- Insert the second haptic inside the eye with a clip while pulling the thread of the second device. By pulling the thread of the device, the second haptic 12' follows the thread to the outside of the eye.
6- Once the lens 10 is inserted in the eye and the two haptics 12 12' connected to each thread (in corresponding devices 50) are outside of the eye, the lens can be pulled and positioned correctly, and the haptics 12 12' can be inserted in the corresponding lodgings (intrascleral tunnels made thereafter). Once the haptics 12 12' are positioned in their intrascleral location, the threads 54 of each device 50 can be cut, so each piece 53 (nozzle) is in the intrascleral position connected to the corresponding haptic. Alternatively, the tip of each haptic can be cut, so that the full device 50 (piece 53 and thread 54) is withdrawn with the cut tips, leaving only the haptics in the intrascleral lodging.

Thanks to device 50, which makes it possible to implement the procedure for inserting intraocular lenses with haptics in a way that is safe for the patient and relaxed for the surgeon, the surgeon avoids having to clamp each haptic of the lens (both the lens and the haptic) located inside the eye with clips and having to move them to their lodging. This also prevents the lens from slipping to the back of the eye. With conventional methods, when clips are used to hold the haptics, they can quickly slip through the clip, and even bend or break and detach from the optical part, causing the lens to fall to the back of the eye. This way, risks of eye lesions and complications of the surgical procedure are avoided.

Furthermore, the invention is not limited to the specific embodiments described, but also covers, for example, variations performed by the expert on the material (for example, regarding selection of materials, dimensions, components, configuration, etc.), without departing from the claims.

## Claims

1. A device (50) to aid the insertion of an intraocular lens (10) in an intraocular cavity in the absence of a capsular bag or sufficient capsular remnants for lens (10) fixation, wherein said intraocular lens (10) is made up of one optical part (11) and two haptics (12, 12'), the device (50) being **characterized in that** it comprises:
- a piece (53) manufactured from a biocompatible material, wherein the piece (53) is configured to receive a portion of haptic (12) in its interior inserted through a first end (56) of the piece (53); and
- a thread (54) made of a biocompatible material attached at one end (52) of said piece (53) opposite the end (56) aimed at receiving the haptic (12), said thread (54) being configured to, in the use of the device, pull and position the optical part (11) of the lens (10) inside the intraocular cavity.

2. The device (50) of claim 1, in which the section of said thread (54) ranges from 0.030 and 0.350 mm in diameter.

3. The device (50) of any of the preceding claims, in which the exterior length of said piece (53) is no greater than 2 mm.

4. The device (50) of any of the preceding claims, in which the part of said piece (53) aimed at receiving a part of the haptic (12) has an interior length of no greater than 1 mm.

5. The device (50) of any of the preceding claims, in which said piece (53) is metallic.

6. The device (50) of any of the preceding claims, in which said piece (53) is a tube designed, in use of the device (50), to crimp a portion of the haptic (12).

7. The device (50) of any of the preceding claims, in which the exterior diameter of said piece (53) ranges between 0.250 and 0.700 mm.

8. The device (50) of any of the preceding claims, in which the interior diameter of said piece (53) ranges between 0.200 and 0.430 mm.

9. The device (50) of any of the preceding claims, comprising a rivet (55) where the thread (54) and the base (52) of the piece (53) join, to reinforce the connection between the thread (54) and the piece (53).

10. An ensemble comprising:
- a first device (50) to aid the insertion of an intraocular lens (10) in an intraocular cavity in the absence of a capsular bag or sufficient capsular remnants for lens fixation (10), according to any of the preceding claims, and
- an intraocular lens (10) comprising an optical part (11), a first haptic (12) and a second haptic (12');
wherein a portion of the free end of said first haptic (12) is designed to be inserted and anchored in said piece (53) of said first device (50).

11. The ensemble of claim 10, further comprising a second device (50) to aid the insertion of an intraocular lens (10) in an intraocular cavity in the absence of sufficient capsular bag or capsular remnants for lens (10) fixation, according to any of claims 1 to 8, in which a portion of the free end of said second haptic (12') is designed to be inserted and anchored in said piece (53) of said second device (50).

12. Use of the device or ensemble, of any of the preceding claims, in a surgical treatment.

13. The device of any of claims 1 to 9, or the ensemble of any of claims 10 to 11, for use in a surgical procedure comprising the insertion and positioning of an intraocular lens in a patient lacking a capsular bag or sufficient capsular remnants for lens fixation (10).

14. A procedure for positioning a 3-piece, foldable intraocular lens (10) comprising an optical part (11) and two haptics (12, 12'), in an intraocular cavity of a patient with no capsular bag or sufficient capsular remnants for lens (10) fixation, using two devices (50) according to any of claims 1 to 9, one for each haptic (12, 12'), comprising:
- Insert a portion of the free end of a first haptic (12) of said lens (10) in a cavity defined by a first device (50), while the optical part (11) of the lens (10) is folded in an injector;
- Close or deform the end of the device (50) defining that cavity until the haptic (12) is anchored to the device (50);
- Insert the free end of the thread (54) of said first device (50) through an incision made in a cornea and thread that free end of the thread (54) out of the sulcus;
- with the help of said injector, insert said lens (10) in the ocular cavity through the incision made in the cornea, such that the free end of the thread (54) remains outside of the patient and, by pulling on it, the first haptic (12) is extracted until it exits the eye, and said second haptic (12') remains outside of the eye;
- Insert a portion of the free end of a second haptic (12') of said lens (10) in a cavity defined by a second device (50), while this second haptic (12') is outside the eye and the optical part (11) of the lens (10) is already injected inside the eye;
- Close or deform the end of the second device (50) defining that cavity until the second haptic (12') is anchored to the second device (50);
- Insert the free end of the thread (54) of said second device (50) through the incision made in the cornea and thread that free end of the thread (54) out of the sulcus;
- Insert said second haptic (12') in the ocular cavity with the help of a clip while pulling on the thread connected to the second haptic (12') from outside the eye, such that the second haptic (12') is extracted from the ocular cavity, following the thread, until it exits the eye;
- Position said optical part (11) of the lens (10) in a desired position in the intraocular cavity by manipulating it from outside of the patient using the two threads (54) connected to the respective haptics (12,12') and affix the two haptics (12, 12') to their respective lodging.
